# EUROPÄISCHE PATENTSCHRIFT

(11) **EP 0 602 164 B1**
(45) Veröffentlichungstag und Bekanntmachung des Hinweises auf die Patenterteilung: **21.08.1996**
(21) Anmeldenummer: 92919613.7
(22) Anmeldetag: 07.09.1992
(51) Int. Cl.: A61B 19/04, A47G 25/90, A45D 44/02, D06F 58/00

(54) **VORRICHTUNG ZUM AN- UND AUSZIEHEN VON HANDSCHUHEN AUS ELASTISCHEM MATERIAL**
DEVICE FOR PUTTING ON AND DRAWING OFF GLOVES MADE OF ELASTIC MATERIAL
DISPOSITIF SERVANT A METTRE OU A ENLEVER DES GANTS EN MATIERE ELASTIQUE

(30) Priorität: 07.09.1991 DE 4129780
(43) Veröffentlichungstag der Anmeldung: 22.06.1994
(73) Patentinhaber: Wella Aktiengesellschaft, D-64274 Darmstadt (DE)
(72) Erfinder: Bauer, Rudolf, 70173 Stuttgart (DE)
(74) Vertreter: Dreiss, Fuhlendorf & Steimle
(86) Internationale Anmeldenummer: EP9202064
(87) Internationale Veröffentlichungsnummer: WO9304637

(56) Entgegenhaltungen:
- WO-A-90/06730
- CH-A- 379 688
- DE-B- 1 222 212
- FR-A- 1 479 808
- US-A- 2 741 410
- US-A- 3 695 493
- US-A- 3 992 221

## Beschreibung

Die Erfindung betrifft eine Vorrichtung zum Anziehen und Ausziehen von Handschuhen aus elastischem Material, die zumindest eine mit Öffnungen versehene Kammer aufweist, und bei der die Öffnungen zum Einbringen der Handschuhe und zum Anlegen derselben mit einer im Manschettenbereich vorgesehenen Auflagefläche beim Ausziehen dienen, und bei der die Kammern mit Einrichtungen zur Erzeugung eines Über- oder Unterdrucks versehen sind.

Derartige Vorrichtungen sind bekannt (vgl. DE 38 42 535 A1, EP 0 347 606 A1, US 36 95 493, US 27 41 410,US 30 67 001, US 19 38 685, US 19 96 377, US 40 02 276, DE 586 997, DE 200 354, DE-OS 26 52 006). Sie sind jedoch insgesamt noch nicht geeignet, eine häufige Verwendung von eng anliegenden elastischen Handschuhen, insbesondere aus sehr dünnem Material, zu ermöglichen, weil die Innenflächen derartiger Handschuhe bereits nach kurzem Gebrauch durch Schweiß sehr feucht werden. Wegen dieser Feuchte kleben die Innenflächen nach auch nur kurzzeitigem Gebrauch nach dem Ausziehen zumindest im Bereich der Finger stark aneinander, so daß sie nicht ein zweites Mal angezogen werden können. Außerdem fühlt sich ein von der vorherigen Benutzung innen schweißnasser Handschuh sehr unangenehm an. Auch dies steht seiner Wiederverwendung im Wege. Dies gilt besonders für sehr dünne Handschuhe, die aber wegen des Gefühls der Hand bei feinmotorischen Bewegungen erwünscht sind. Schließlich wünscht man die Wiederverwendbarkeit, um das Entsorgungsproblem gebrauchter Handschuhe so gering wie möglich zu halten. Das ist z.B. bei Friseuren ein großes Problem, weil z.B. beim Haarfärben und Dauerwellen mit Unterbrechungen dreimal Handschuhe benötigt werden.Die nur einmalige Verwendung von Handschuhen wäre ökologisch nicht zu vertreten.

Aufgabe der Erfindung ist es daher, eine Vorrichtung der eingangs genannten Art derart weiterzubilden, daß ein nach Benutzung an seiner Innenfläche durch Schwitzen naß gewordener Handschuh einfach ausgezogen, getrocknet und wieder angezogen werden kann, und dieses mehrfach.

Erfindungsgemäß wird diese Aufgabe dadurch gelöst, daß eine Einrichtung zur Trocknung der Innenflächen der Handschuhe durch Anströmen der Innenflächen mit einer Strömung, die von den genannten Einrichtungen zur Erzeugung eines Über- oder Unterdrucks erzeugt wird.

Vorteilhafte Weiterbildung der Erfindungen ergeben sich aus den Unteransprüchen.

Mit einem derartigen Gerät ist es möglich, nach dem Ausziehen einen innen schweißnassen Handschuh binnen kurzer Zeit - erfahrungsgemäß etwa innerhalb von eineinhalb Minuten - wieder zu trocknen. Auch elastische Handschuhe aus sehr dünnem Material können daher mehrfach wiederverwendet werden. Die Anwendung eines erfindungsgemäßen Gerätes kommt vor allem im Friseurbereich infrage, in dem der Schutz der dort tätigen Personen vor Allergien, die durch den Kontakt mit Haarbehandlungsmitteln entstehen, zunehmend ein großes Problem darstellt. Bei Trocknung gemäß der Erfindung können Handschuhe, aus hygienischen Gründen einer Person zugeordnet, zumindest einen Tag lang beliebig oft verwendet werden. Weitere Anwendungen ergeben sich im medizinisch/klinischen, im Nahrungsmittel- und im chemischen Bereich.

Die erfinderische Idee liegt also darin, die Innenfläche des Handschuhs anschließend an dem mit Hilfe des Unterdrucks in der Kammer unterstützten Ausziehvorgang gerätemäßig zu trocknen und dafür geeignete Maßnahmen vorzusehen. Bei einem Ausführungsbeispiel verbleibt der Handschuh in der nach innen in die Kammer hineingezogenen Stellung; zur Trocknung der Innenfläche des Handschuhs wird von oben Luft in das Innere des Handschuhs eingeblasen; dabei kann die Ausbildung so getroffen werden kann, daß ein Luftkreislauf entsteht. Bei anderen Ausführungsbeispielen wird der Handschuh nach außen umgestülpt. Die Innenfläche des Handschuhs, die naß ist und getrocknet werden muß, wird dann durch diesen Umstülpvorgang bei der Trockung die Außenfläche. Sie wird dann von außen angeströmt.

Die Ausführungsbeispiele der Erfindung und ihrer vorteilhaften Weiterbildungen werden im folgenden anhand der beigefügten Zeichnungen beschrieben. Es stellen dar:
- Figur 1: ein Ausführungsbeispiel;
- Figur 2: eine Ansicht in Richtung der Pfeile II-II ihn Figur 1;
- Figur 3: eine Draufsicht auf das Gerät nach Figur 2, entsprechend den Pfeilen III-III;
- Figur 3a: eine schematische Darstellung der Lage der Öffnungen 19,20,60-66 in Platte 3 zu den Öffnungen 22,23,50-56 und Schlitz 57 in Platte 21 bei verschobener Platte 21;
- Figur 4: einen Ring 15 zur Verstärkung des Handschuhs;
- Figur 5: einen Handschuh 16, dessen Manschette über den Ring 15 gezogen ist;
- Figur 6: eine Teilansicht des Ausführungsbeispiels im Schnitt, entsprechend Figur 1, jedoch mit aufgesetztem Deckel 30;
- Figur 7: ein zweites Ausführungsbeispiel im ersten Betriebsmodus;
- Figur 8: das zweite Ausführungsbeispiel im zweiten Betriebsmodus;
- Figur 9: die schematische Darstellung eines dritten Ausführungsbeispiel im ersten Betriebsmodus;
- Figur 10: die Draufsicht auf das Ausführungsbeispiel nach Figur 9 im ersten Betriebsmodus;
- Figur 11: das dritte Ausführungsbeispiel im zweiten Betriebsmodus.

Die Vorrichtung weist einen Wagen 1 auf, der durch vertikale Streben 2 gebildet wird, die durch eine obere horizontale Platte 3 und eine untere horizontale Platte 4 verbunden sind. Zwischen der oberen Platte 3 und der unteren Platte 4 sind zwei Rohre 5,6 aus Plexiglas stehend eingebracht. Die Rohre 5,6 bilden zusammen mit den Platten 3,4, durch die sie oben und unten begrenzt werden, Kammern. In die untere Platte 4 sind Sauggebläse 7,8 dicht eingepaßt, die innerhalb der Kammern Unterdruck erzeugen. Die Sauggebläse 7,8 sind mit - durch elektrische Widerstandsdrähte angedeuteten - Heizungen 9,10 versehen, die von einem Schalter 11 einschaltbar sind. Die von den Sauggebläsen 7,8 abgesaugte - und ggf. durch die Heizungen 9,10 erhitzte - Luft wird über Abluftleitungen 12,13 abgeführt. Die Sauggebläse 7,8 können durch einen Fußschalter 14 ein- bzw. ausgeschaltet werden.

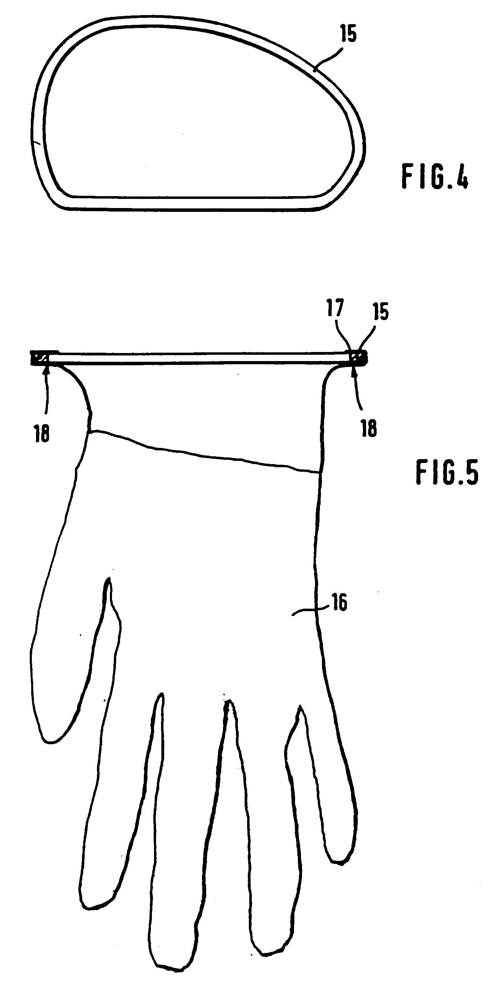

Um mittels des Gerätes Handschuhe 16 anziehen zu können, werden diese mit ihrem Manschettenbereich, d.h. ihrem offenen Ende, das meist mit einem kleinen Wulst 17 versehen ist, über einen starren Ring 15 gezogen, der vorzugsweise aus flachem Material ausgestanzt ist (vgl. Figuren 4,5).

Die Kontur des Ringes 15 ist so bestimmt, daß man mit einer Hand H bequem hindurchgreifen kann. Die Kontur ist gleichzeitig in ihren Abmessungen auch etwas größer als der nicht aufgeweitete Durchmesser des Handschuhs 16, so daß sich im Anschluß an die Stelle, an der der Rand des Handschuhs 16 den Ring 15 umgibt, eine elastische Einschnürung ergibt, durch die eine Auflagefläche 18 entsteht.

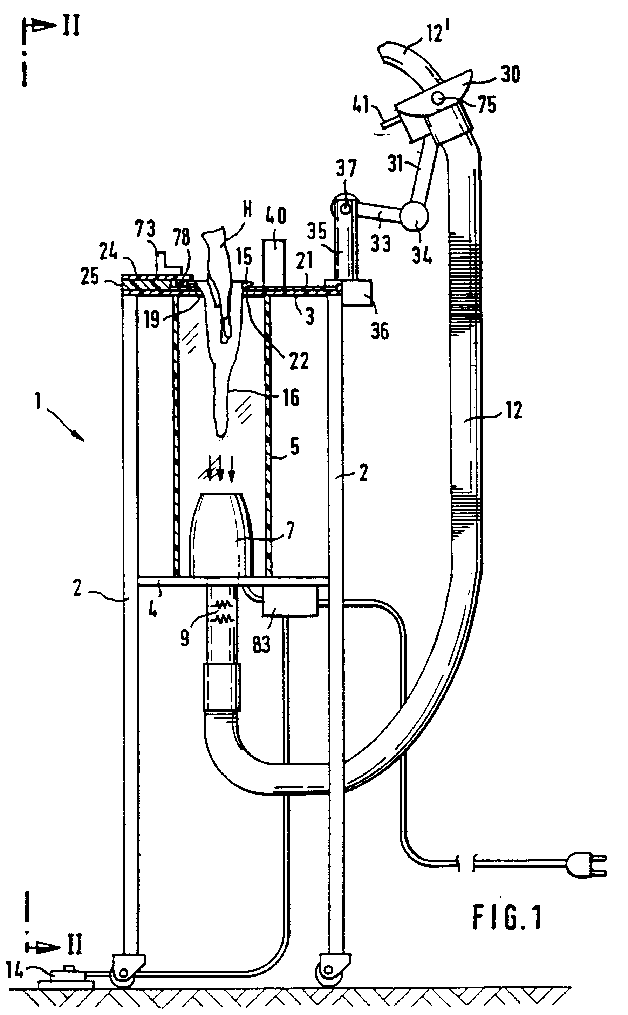

Wie aus Figur 1, 3 und 6 zu ersehen, weist die Platte 3 an ihrer Oberseite zwei Öffnungen 19,20 auf. Oberhalb der Platte 3 befindet sich verschiebbar eine weitere Platte 21. Diese Platte 21 ist ebenfalls mit Öffnungen, nämlich den Öffnungen 22,23, versehen.

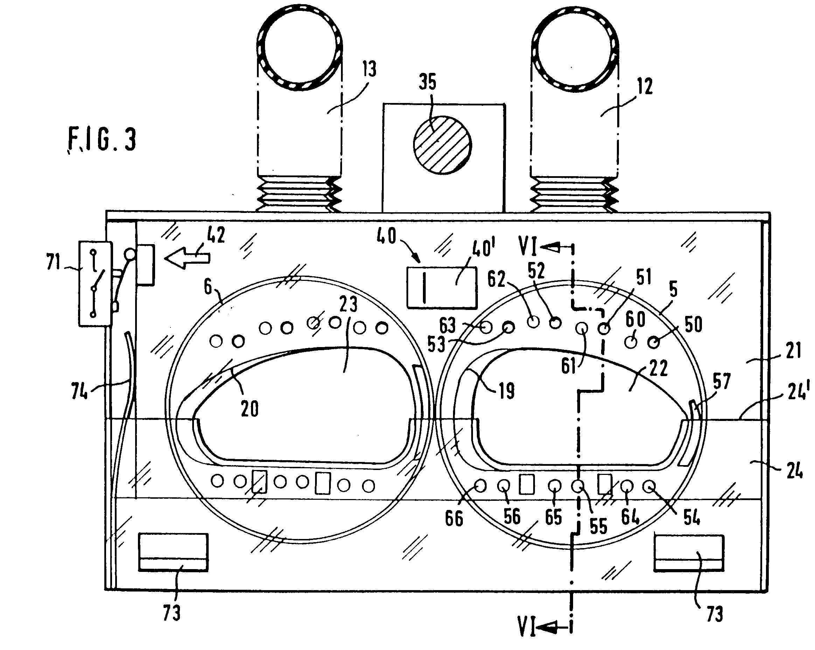

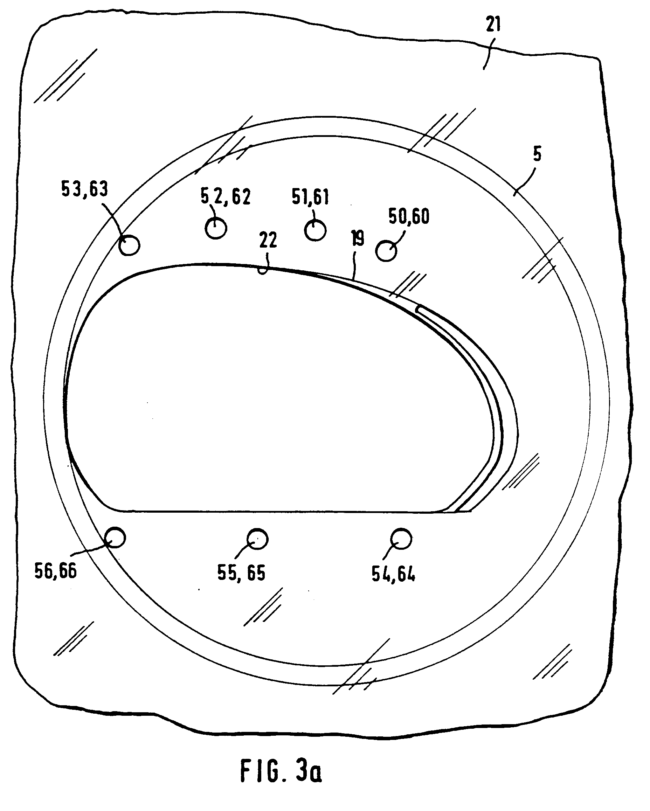

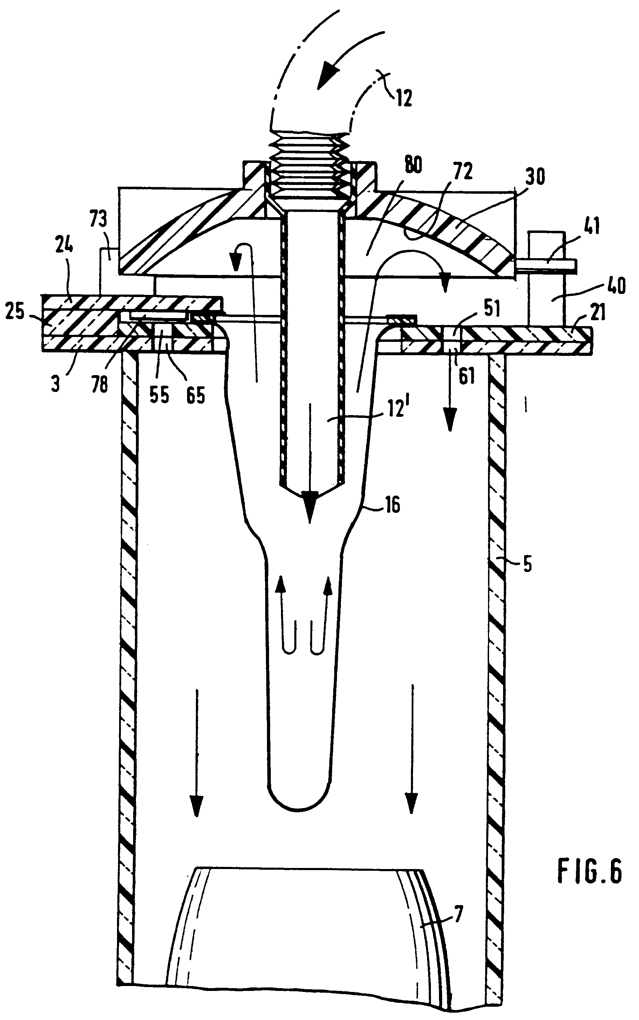

Wie aus Figur 3 deutlich wird, sind die Öffnungen 22,23 in den Abmessungen ihrer Kontur etwas geringer als die Ringe 15, so daß die sich beim Überstreifen eines Handschuhes 16 unterhalb des Ringes 15 ausbildende Auflagefläche 18 auf dem äußeren Rand der Öffnungen 22,23 der Platte 21 auf- bzw. anliegen kann (vgl. Figuren 1,6). Die Öffnungen 19,20 in der Platte 3 sind teilweise deckungsgleich mit den Öffnungen 22,23 in der Platte 21; jedoch sind die Öffnungen 19,20, wie aus den gestrichelten Linien in Figur 3 zu ersehen, nach links hin etwas weiter.

Oberhalb der Platte 21 befindet sich in einem Abstand von ca. 5 mm eine Niederhalteplatte 24. Sie wird von einer Leiste 25 getragen. Die Platte 24 weist einen Rand 24' auf (vgl. Figur 3), der die Öffnungen 22,23 abschnittsweise etwas überragt.

Beim **Anziehen** eines Handschuhes 16 geht man also wie folgt vor: Man streift die Manschettenbereiche der Handschuhe 16 über zwei Ringe 15, wie dies bereits anhand von Figur 5 beschrieben worden ist. Dann legt man die Handschuhe 16 mit ihren Auflageflächen 18, die sich entlang des Umfangs der Ringe 15 ausgebildet haben, auf die Ränder der Öffnungen 22,23 in der Platte 21, und zwar derart, daß die lockeren Handschuhe 16 in das Innere der Rohre 5,6 hineinfallen. Zur Festlegung der Lage der Handschuhe 16 dienen die Anschläge 78, die an der Niederhalteplatte 24 angeordnet sind. Dann betätigt man den Fußschalter 14 und schaltet damit die Sauggebläse 7,8 ein. Es bildet sich ein Unterdruck in den durch die Rohre 5,6 gebildeten Kammern aus, der die elastischen Handschuhe 16 euterartig aufweitet. Man kann nun mit den Händen von oben - wie aus Figur 1 ersichtlich - bequem in die Handschuhe 16 hineinfahren und sie somit anziehen. Dann schaltet man mit dem Fußschalter 14 die Sauggebläse 7,8 wieder aus. Man bewegt sich dann mit den Handschuhen 16, in denen die Ringe 15 verbleiben, aus den Öffnungen 22,23 der Platte 21 und damit der Öffnungen 19,20 der Platte 3 heraus. Dabei achtet man darauf, daß der durch einen Ring 15 verstärkte Manschettenbereich der Handschuhe 16 zunächst nach kurzem Anheben aus dem Bereich unter dem Rand 24' der Niederhalteplatte 24 horizontal herausbewegt wird.

Das **Ausziehen** der Handschuhe 16 erfolgt analog. Man streckt die Hände mit Handschuhen 16, an denen die Ringe 15 noch angebracht sind, durch die Öffnungen 22,23 in Platte 21 und damit auch durch die Öffnungen 19,20 in Platte 3 hindurch. Dann wird der Ring 15 mit Auflagefläche 18 auf den Rand entlang des Umfangs der Öffnungen 22,23 auf Platte 21 aufgelegt und horizontal mit seinem in Fig. 3 unteren Teil unter dem Rand 24' der Niederhalteplatte 24 geschoben. Der Rand 24' hält den Handschuh 16 mit dem den Ring 15 umgebenden Manschettenbereich zurück, während man sich mit der Hand H aus den somit zurückgehaltenen Handschuhen 16 herausbewegt. Bevor man mit der Herausbewegung beginnt, wird der Fußschalter 14 betätigt und damit die Sauggebläse 7,8 eingeschaltet, um den unterdruck im Innenraum der durch die Rohre 5,6 gebildeten Kammern zu erzeugen. Bewegt man die Hände H aus den Handschuhen 16 heraus, so werden diese dabei umgestülpt; sie werden jedoch, sobald die Hand H vollständig aus den Handschuhen 16 herausgezogen ist, wieder in den Innenraum der Rohre 5,6 hineingezogen.

Für die **Trocknung** des Inneren der Handschuhe 16, nachdem sie ausgezogen worden sind, ist folgendes vorgesehen:

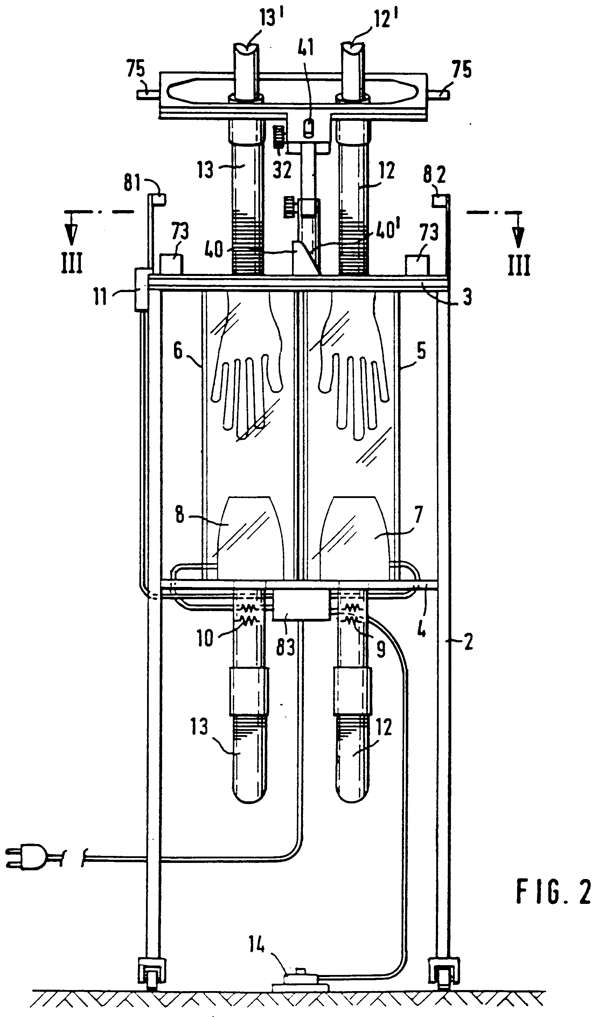

An dem Wagen 1 befindet sich beweglich ein Deckel 30. Zu diesem hin und durch diesen hindurch sind die Abluftleitungen 12,13 der Sauggebläse 7,8 geführt. Die offenen Enden 12',13' der Abluftleitungen 12,13 bilden - bei eingeschalteten Heizungen 9,10 - Warmluftaustrittsrohre (vgl. auch Figur 2). Sie ragen ca. 10 cm über den Deckel 30 hinaus. Der Deckel 30 bildet zusammen mit den Warmluftaustrittrohren 12', 13' und den Heizungen 9,10 die Warmluftzufuhr-Einrichtung. Dabei kann die Luftzufuhr und Erwärmung auch anderweitig erfolgen, wenngleich die Nutzung und Erwärmung der Abluft der Sauggebläse 7,8 besonders vorteilhaft ist. Der Deckel 30 ist an einem ersten Hebel 31 angelenkt. Die Anlenkung kann mit Hilfe einer Rändelschraube 32 (vgl. Figur 2) festgestellt werden. Der Hebel 31 ist seinerseits an einem weiteren zweiten Hebel 33 drehbar befestigt, wobei diese Drehverbindung mit Hilfe der Rändelschraube 34 festgestellt werden kann. Der zweite Hebel 33 ist seinerseits drehbar an dem dritten Hebel 35 angelenkt. Der Hebel 35 ist an einer Platte 36 befestigt. Die Platte 36 ist mit der Platte 3 fest verbunden. Die Rändelschrauben 34 und 32 sind in einer Stellung fest angezogen, so daß die Relativstellung des Hebels 31 zum Deckel 30 und die der Hebel 31,33 zueinander fest ist. Die durch die Hebel 31,33 und den Deckel 30 gebildete Einheit kann um die Drehverbindung 37 zwischen den Hebeln 35 und 33 geschwenkt werden. Am Deckel befinden sich ferner Handgriffe 75.

Befinden sich Handschuhe 16, die gerade ausgezogen worden sind, in der Vorrichtung, wie aus Figur 1 ersichtlich, so wird, wenn man die Bedienungsperson ihre dort eingezeichnete Hand H herausgezogen hat, der Deckel 30 über die Handschuhe 16 geschwenkt, derart, daß der flächige Deckel 30 die vom Ring 15 aufgehaltenen offenen Enden der Handschuhe 16 überdeckt, wobei die durch die offenen Enden der Abluftleitungen 12,13 gebildeten Warmluftaustrittsrohre 12',13' nunmehr in die Handschuhe 16 hineinragen. Vorzugsweise sind die Warmluftaustrittsrohre 12',13' elastisch, so daß sie leicht ausweichen können, wenn sie auf die Ränder der Öffnungen 22,23 auftreffen. Die Enden der Warmluftaustrittsrohre 12',13' sind ferner glockenartig ausgeschnitten, um eine Verteilung der ausströmenden Warmluft zu erreichen. Sie bilden in die Handschuhe eintauchende Trocknungsdüsen. Dabei wird wieder der Fußschalter 14 betätigt, so daß die Handschuhe 16 von innen durch die aus den Warmluftaustrittsrohren 12',13' ausströmende Luft an- und aufgeblasen werden. Die Aufdehnung wird dadurch unterstützt, daß in den Rohren 5,6 ja infolge des eingeschalteten Zustands der Sauggebläse 7,8 Unterdruck herrscht.

Gleichzeitig laufen noch folgende Vorgänge ab:

Auf der Platte 21, die auf der Platte 3 verschiebbar ist, befindet sich ein Nocken 40, der - wie aus Figur 2 ersichtlich - eine schräge Fläche 40' aufweist. Beim Schwenken des Deckels 30 in die Position oberhalb der Handschuhe 16 (Figur 6) trifft nun der Stift 41, der am Deckel 30 angebracht ist, auf die schräge Fläche 40' und drückt diese nach links (in Figur 2). Da der Nocken 40 mit der Platte 21 fest verbunden ist, erfolgt darauf eine Verschiebung der Platte 21 nach links, wie durch den Pfeil 42 in Figur 3 angedeutet, und zwar gegen die Kraft der Feder 74.

In der Platte 21 sind neben den bereits beschriebenen Öffnungen 22,23 entlang deren Umfangs weitere Öffnungen vorgesehen, nämlich die Öffnungen 50,51,52,53,54,55,56, sowie ein sichelförmiger Schlitz 57. Entsprechende Öffnungen und ein entsprechender Schlitz umgeben die Öffnung 23. Der Übersichtlichkeit halber sind dort jedoch keine Bezugszeichen gezeichnet. Die Öffnungen 54,55,56 sind in Figur 3 gestrichelt eingezeichnet, weil sie in der Draufsicht unter der Niederhalteplatte 24 liegen.

In der unterhalb der Platte 21 liegenden Platte 3 befinden sich, wie bereits erwähnt, die Öffnungen 19,20. Es sind auch in Platte 3 noch weitere Öffnungen vorgesehen, nämlich die Öffnungen 60,61,62,63,64,65,66. Sie alle sind, weil hinter bzw. unter der Platte 21 liegend, ebenfalls gestrichelt eingezeichnet. Die Öffnungen 60,...,66 in Platte 3 sind gegenüber den Öffnungen 50,...56 in Platte 3 alle um denselben Betrag in Richtung des Pfeiles 42 versetzt.

Wird nun die Platte 21 infolge der Herabbewegung des Deckels 30 und des Eingriffes des Stiftes 41 mit dem Nocken 40 nach links verschoben, so folgt daraus, daß, da die Platte 3 ja nicht mit verschoben wird, die Öffnungen 50,60, die Öffnungen 51,61, usw., schließlich die Öffnungen 56,66 miteinander fluchten. Der Schlitz 57 in Platte 21 öffnet sich direkt in die darunterliegende Öffnung 19 in der Platte 3. Diese Situation ist schematisch für diesen Teilbereich von Figur 3 nochmal in Figur 3a herausgezeichnet. Die miteinander fluchtenden Öffnungen 50,60';....bis 56,66, sowie der mit der Öffnung 19 fluchtende Schlitz 57 stellen also Öffnungen der durch die Rohre 5,6 gebildeten Kammern nach oben durch die Platten 3 und 21 hindurch zu dem Bereich 80 zwischen Deckel 30 und Platte 21 dar; dies jedoch unter der Voraussetzung, daß die Platte 21 in Richtung des Pfeiles 42 entsprechend verschoben ist. Die miteinander in Verbindung stehenden Öffnungen 50,60,...56,66 und 57,19 bzw. 57,20 ermöglichen also, daß die Warmluft, die aus dem Inneren der Handschuhe 16 in den Bereich 80 zurückströmt, von dort in die Kammern 5,6 übertritt. Dadurch wird der Unterdruck in den Kammern 5,6 bei entsprechender Dimensionierung der Öffnungen etwa um die Hälfte reduziert.

Bei Verschiebung der Platte 21 in Richtung des Pfeils 42 wird ferner noch der Schalter 71 geschlossen. Dieser dient dazu, die Heizungen 9,10 an den Sauggebläsen 7,8 einzuschalten. Daraus folgt, daß sich nun - und zwar mit durch die Heizungen 9,10 angewärmter Luft - der in Figur 6 schematisch dargestellte geschlossene Strömungskreislauf ergibt: aus den Warmluftaustrittsrohren 12',13' tritt die Warmluft in das Innere der Handschuhe 16 ein. Sie verwirbelt dort, insbesondere in den Fingerbereichen der Handschuhe 16; sie strömt entlang des äußeren Bereichs des Innenraums der Handschuhe 16 nach oben zurück und von dort in den Bereich 80 unterhalb des Deckels 30. Der Deckel 30 ist innen gewölbt und mit einer konkaven inneren Strömungsumlenkfläche 72 versehen.

Die Strömung wird an dieser Strömungsumlenkfläche 72 umgelenkt und tritt dann durch die Öffnungen 50,60, usw. in die durch die Rohre 5,6 gebildeten Kammern ein, von denen die Strömung durch das Sauggebläse 7 abgezogen und wieder den Warmluftaustrittsrohren 12',13' zugeführt wird. Es entsteht also ein Warmluftkreislauf, der gleichzeitig von innen (durch Überdruck) und außen (durch Unterdruck) die Handschuhe 16 aufweitet und sie dabei von innen mit Warmluft anströmt und damit schnell trocknet. Auch außen werden die Handschuhe 16 umströmt und damit getrocknet, z.B. nachdem sie vorher (angezogen) gewaschen werden. Danach können die Handschuhe 16 wieder angezogen werden. Die Reduzierung des Unterdrucks in den Kammern 5,6 bei Verschiebung der Platte 21 und dadurch bewirkter Freigabe der Öffnungen hält die Handschuhe für die Trocknung genügend aufgeweitet. Die gegenüber dem euterartigen Aufblasen zum Anziehen reduzierte Aufweitung in dieser Warmluft-Trocknungsphase sichert die Formhaltigkeit der Handschuhe.

Es ist eine Zeitschaltuhr 83 vorgesehen, die bei geschlossenem Deckel 30 und somit geschlossenem Schalter 11 die Heizungen 9,10 stets nach einer gewissen Zeit wieder abschaltet, um eine Übertrocknung und Materialschädigung zu vermeiden. Ist die Trocknung erfolgt, hebt man den Deckel 30 wieder an. Die Platte 21 geht in die Ausgangsstellung zurück. Der Unterdruck in den Kammern 5,6 steigt wieder an. Die Handschuhe 16 werden verstärkt aufgeweitet und stehen zum erneuten Anziehen bereit.

Auf der Oberseite der Niederhalteplatte 24 sitzen Winkel 73, die zur Abstützung und Festlegung des Deckels 30 dienen.

Zur Erzielung der beschriebenen Strömung muß der Deckel 30 durchaus nicht dicht auf der Oberseite der Platte 21 sitzen. Der gezeigte Abstand von 1 - 2 cm kann durchaus vorhanden sein. Dadurch, daß an den Öffnungen 50,60,... zum Bereich 80 hin stets Unterdruck wirksam ist sowie ferner dadurch, daß die Strömung durch die Strömungsumlenkfläche 72 in Richtung auf die Öffnungen 50,60,... umgelenkt wird, sowie schließlich dadurch, daß ferner durch die Handschuhe 16 nach oben zurückströmende Warmluft im Bereich 80 unterhalb des Deckels 30 stets ein gewisser Überdruck herrscht, ist die Strömung sichergestellt. Es ist dabei stets gewährleistet, daß der Druck im Inneren des Handschuhs 16 etwas höher als der in den durch die Rohre 5,6 gebildeten Kammern ist, so daß die Handschuhe 16 stets etwas aufgeblasen bleiben.

Man kann die Handschuhe 16 auch von vorneherein ausbilden, daß sie eine Auflagefläche 18 aufweisen, etwa durch einteilige Herstellung mit einem flachen Ring 15.

Die Heizungen 9,10 - etwa durch Widerstandsdrähte gebildet - sind so ausgelegt, daß die Temperatur der Luftströmung auf eine mit dem Material der Handschuhe 16 noch sicher verträgliche Temperatur gebracht wird.

Man kann den Fußschalter 14, der zum Ein- und Ausschalten der Sauggebläse 7,8 dient, durch eine Infrarotlichtschraube 81,82 ersetzen, so daß beim Einführen der Hände automatisch ein Einschalten erfolgt.

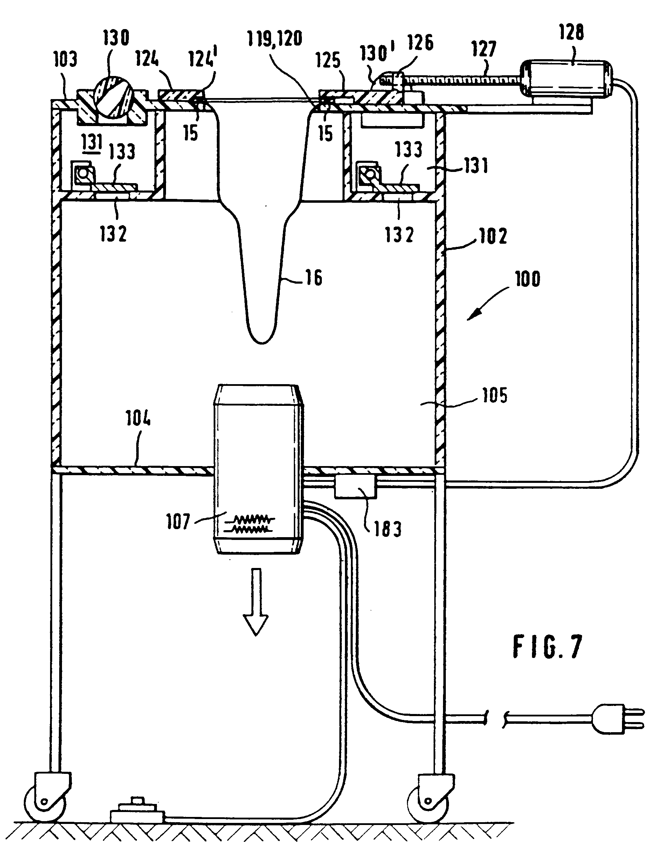

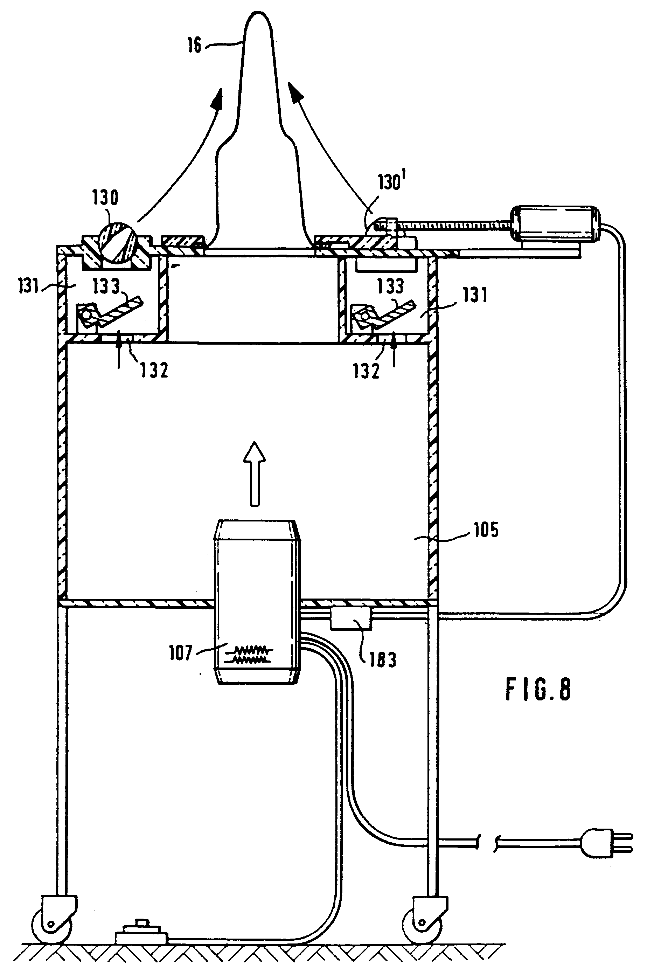

Bei dem Ausführungsbeispiel nach den Figuren 7 und 8 ist ein Wagen 100 vorgesehen, der eine obere horziontale Platte 103 und eine untere horizontale Platte 104 aufweist, die zusammen mit Seitenplatten 102 eine geschlossene Kammer 105 bilden. In der unteren horizontalen Platte 104 befindet sich ein umschaltbares Gebläse 107, das im ersten Betriebsmodus (Figur 7) aus der Kammer 105 Luft absaugt und in dieser somit Unterdruck erzeugt und im zweiten Betriebsmodus (Figur 8) in umgekehrter Richtung Luft transportiert und somit in der Kammer 105 Überdruck erzeugt.

In der oberen horizontalen Platte 103 befinden sich, analog zu der Darstellung beim ersten Ausführungsbeispiel, zwei Öffnungen 119,120, die zur Aufnahme von zwei Handschuhen 16 dienen. Aus den Figuren 7,8 ist nur einer ersichtlich, da die Darstellung der Figur 1 entspricht. Auf der oberen horizontalen Platte 103 wiederum befindet sich eine Niederhalteplatte 124, deren Rand 124' so konturiert ist wie der Rand 24' der Niederhalteplatte 24 beim ersten Ausführungsbeispiel, so daß er etwa die Hälfte des Ringes 15, der in den Handschuh 16 eingesetzt ist, umgreift. Dem Niederhalter 124 gegenüber sind einer oder mehrere verschiebbare Niederhalter 125 vorgesehen. Ein Niederhalter 125 weist an seinem einen Ende einen Flansch 126 auf, in diesem Flansch ist eine mit einem Innengewinde versehene Bohrung vorgesehen, die von einer Gewindestange 127 durchsetzt wird, die von einem Elektromotor 128 in beiden Drehrichtungen antreibbar ist. Der Niederhalter 125 kann also bei entsprechender Drehrichtung des über den Ring 15 geschoben werden, (Verschiebung nach links in die in Figur 7 gezeigte Endstellung). Dann hält der Niederhalter den in den Handschuh 16 eingesetzten Ring 15, wie in Figur 7 gezeigt; schaltet man den Elektromotor 128 um, so daß er sich in anderer Richtung dreht, so wird der Niederhalter 125 in Figur 7 nach rechts weggezogen, so daß dann der Handschuh 16 entnommen werden kann. Die für die Hin- und Her- Verschiebebewegung des Niederhalters 125 erforderlich Führung desselben in der oberen horizontalen Platte 103 ist der Einfachheit halber nicht dargestellt. Die Umschaltung dese Elektromotors 83 ist in der Steuerung 183 integriert.

In der Platte 103 befinden sich um die Öffnungen 119,120 mehrere verschwenkbare Luftdüsen 130,130'. Ihre Luftdurchtrittskanäle stehen nach oben mit der Atmosphäre in Verbindung; nach unten stehen sie mit einer um die Öffnungen 119, 120 herum angeordneten Kammer 131 in Verbindung, die ihrerseits mit der Kammer 105 über Öffnungen 132 in Verbindung steht. Die Öffnungen 132 sind durch Ventilklappen 133 verschließbar. Sie liegen von oben auf den Öffnungen 132 auf, so daß sie in dem in Figur 7 dargestellten Betriebsmodus, in dem in der Kammer 105 unterdruck herrscht, geschlossen sind; in dem in Figur 8 dargestellten Betriebsmodus jedoch, in dem in der Kammer 105 Überdruck herrscht, sind die Ventilklappen 133 jedoch offen.

Die Funktionsweise des in Figur 7 und 8 dargestellten Ausführungsbeispiels ist folgende:

Zunächst werden, wie beim ersten Ausführungsbeispiel beschrieben, die beiden Handschuhe 16 in die Öffnungen 119,120 eingesetzt, wobei der Ring 15 von der Person, die die Handschuhe ausziehen will, unter den Rand 124' des Niederhalters 124 geschoben wird. Dann wird das Gerät eingeschaltet, wobei dies zweierlei bewirkt: Erstens wird der Elektromotor 128 so eingeschaltet, daß er sich derart dreht, daß die Niederhalter 125 in die in Figur 7 gezeigte Stellung verbracht werden. Ist dies erreicht, so schaltet sich der Elektromotor 128 selbsttätig ab. Zweitens wird das Gebläse 107 derart eingeschaltet, daß es in der Kammer 105 Unterdruck erzeugt. Damit werden also die Handschuhe 16 von dem Unterdruck in der Kammer 105 so gehalten, daß ihr Ausziehen derselben erleichtert wird. Wenn also jemand, der mit angezogenen Handschuhen in die Öffnungen 119,120 eingreift, seine Hände zurückzieht, unterstützt der Unterdruck in den Kammern 105 das Ausziehen der Handschuhe, und zwar selbst dann, wenn ihre Innenflächen feucht sind und deshalb an den Händen kleben und beim Ausziehvorgang sich nach oben umstülpen. Auf jeden Fall stellt sich unmittelbar nach dem Herausziehen der Hände aus den in die Öffnungen 119,120 eingesetzten Handschuhe 16 sehr schnell die in Figur 7 gezeigte Stellung der Handschuhe 16 ein.

Nach einem gewissen Zeitablauf erfolgt, gesteuert durch die Steuerung 183, eine Umschaltung der Strömungsrichtung des Gebläses 107, so daß sich der in Figur 8 gezeigte Betriebsmodus einstellt, bei dem Überdruck in der Kammer 105 herrscht. Infolgedessen stülpt sich der Handschuh 16 nach außen. Ferner klappen die Ventilklappen 133 nach oben und geben damit die Öffnungen 132 frei, so daß die unter Überdruck stehende Luft aus der Kammer 105 in die Kammer 130 übertreten und von dort aus den Luftdüsen 130,130' austreten kann. Die Luftdüsen 130,130' werden so gestellt, daß sie die Außenseite der Handschuhe 16 anströmen. Dabei ist durch die Dimensionierung der Öffnungen 132 dafür gesorgt, daß der Druckabfall durch das Austreten von Druckluft nicht verhindert, daß der Handschuh 16 in der in Figur 8 gezeigten Stellung verbleibt. Auf diese Weise erfolgt eine sehr schnelle Trocknung. Man kann dann vorsehen, daß die Zeitschaltuhr 83 wiederum nach einer gewissen für die Trocknung sicher ausreichenden Zeit das Gebläse 107 umschaltet, so daß der Handschuh wieder in die in Figur 7 gezeigte Stellung gebracht wird. Dann wird der Elektromotor wieder angeschaltet und zwar in einer solchen Drehrichtung, daß der Niederhalter 125 in den Figuren 7,8 nach rechts zurückgezogen wird. In dieser Stellung sind die Handschuhe wieder für ein erneutes Anziehen durch einfaches Hineinstrecken der Hände bereit.

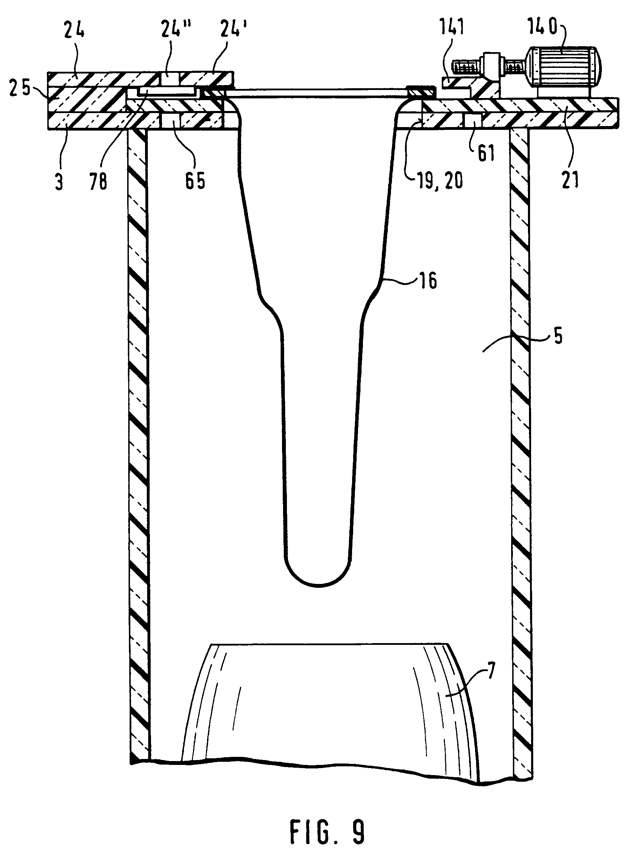

Die Figuren 9 bis 11 zeigen ein drittes Ausführungsbeispiel, das aus dem ersten Ausführungsbeispiel abgeleitet ist, und zwar insoweit, als die Abluftleitungen 12,13 und der Deckel 30 mit den zugeordneten Teilen weggelassen sind. Sofern dieselben Bezugszeichen wie in den Figuren 1 bis 6 verwendet werden, bezeichnen sie auch dieselben Teile.

Nach Figur 9 sitzt also auch, wie beim Ausführungsbeispiel nach den Figuren 1 bis 6, auf der oberen horizontalen Platte 3 eine Leiste 25 und darauf eine Niederhalteplatte 24, deren Rand 24' gegenüber der Platte 3 einen gewissen Abstand hat, so daß der durch den Ring 15 verstärkte Manschettenbereich der Handschuhe 16 dazwischen gelegt werden kann; dies kann allerdings nur etwa mit der Hälfte dieses Manschettenbereiches geschehen (wie auch bei den Figuren 1 bis 6; vgl. dazu insbesondere die Figuren 3 und 3a sowie die dazugehörigen Erläuterungen). In dieser Situation, die in Figur 9 dargestellt ist, wird also zunächst der Handschuh locker eingelegt. Dann wird das Gebläse 7 so geschaltet, daß es in den Kammern 5,6 unterdruck erzeugt. Damit wird der Handschuh 16 in der gezeigten Stellung gehalten und man kann mit der Hand hineinschlüpfen, und den Handschuh also anziehen.

Man nimmt dann zum Arbeiten die Hand mit dem angezogenen Handschuh heraus, wobei man den verstärkten Manschettenbereich des Handschuhs 16 unter dem Rand 24' leicht hervorziehen kann.

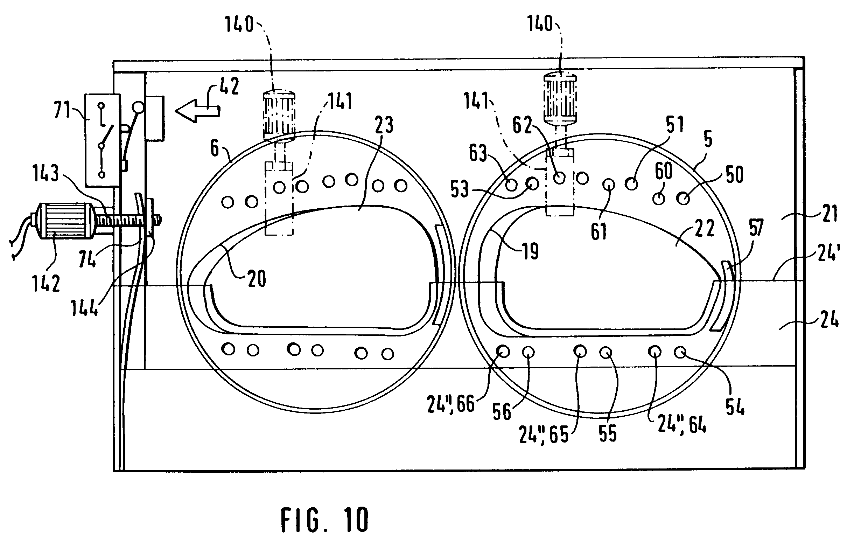

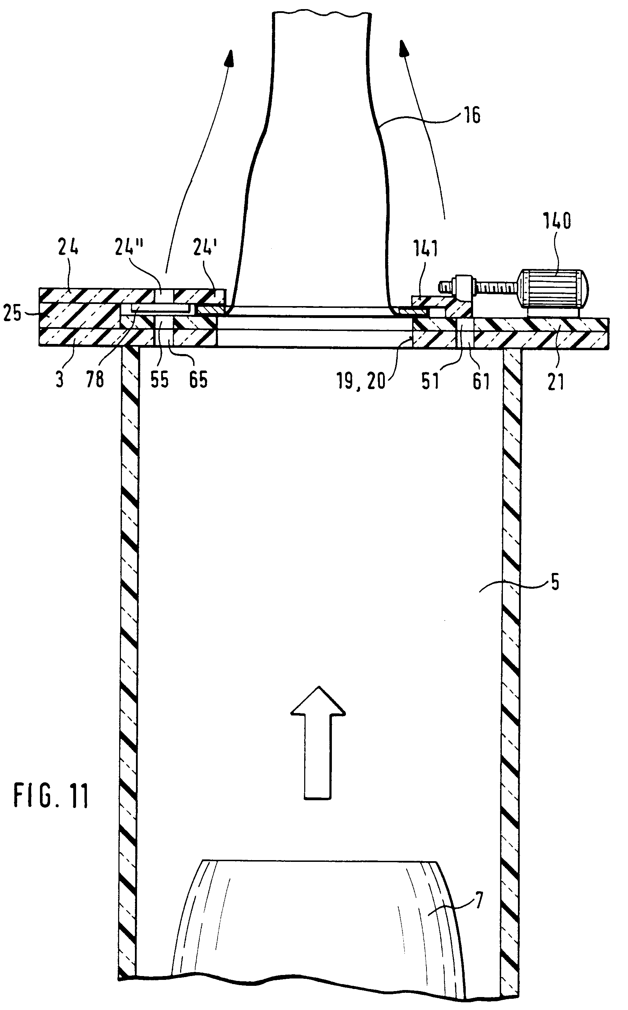

Zum Ausziehen der Handschuhe greift die behandschuhte Hand wieder in die Öffnungen 19,20 in der Platte 3 hinein. Bei Betätigen des Fußschalters 14 werden die Sauggebläse 7,8 so eingeschaltet, daß in der Kammer 105 Unterdruck erzeugt wird. Dann kann man den Handschuh 16 ausziehen. Nach einer dafür ausreichenden Zeit wird der Elektromotor 140 von der Steuerung 183 automatisch eingeschaltet. Er verschiebt die beiden Niederhalter 141 nach links, so daß sich die in Figur 11 gezeigte Position desselben ergibt. Damit übergreifen die Niederhalter, von denen je einer einer Öffnung 19,20 zugeordnet ist (vgl. Figur 10), denjenigen Teil der durch den Ring 15 verstärkten Manschette des Handschuhs 16, der dem Teil, der durch den Rand 24' niedergehalten wird, gegenüber liegt. Gleichzeitig verschiebt der Elektromotor 142 die Platte 21 in Figur 10 nach links, und zwar dadurch, daß er eine Gewindestange 143 dreht, die in eine Gewindebohrung im Flansch 144 eingreift, der an der Platte 21 angebracht ist. Damit werden nun also, wie wiederum u.a. aus Figur 11 zu ersehen, die Öffnungen 50-56 in der Platte 21 mit den Öffnungen 60-66 in der Platte 3 zur Deckung gebracht, so u.a. die Öffnungen 61,51 und 65,55. Oberhalb der Öffnungen 64,65,66 in der Platte 3 befinden sich in der Niederhalteplatte 24 Öffnungen 24''. Ist die Verschiebung der Niederhalter 141 und der Platte 21 erfolgt, so wird ebenfalls von der Steuerung 183 die Richtung des Sauggebläses 7 umgeschaltet, so daß nunmehr in der Kammer 5 Überdruck erzeugt wird. Dadurch wird der Handschuh 16, wie in Figur 11 gezeigt, nach außen umgestülpt. Gleichzeitig strömt durch die miteinander fluchtenden Öffnungen 50,60;...;55,65,24'';56,66 Luft aus der Kammer 105 nach oben aus und trocknet die nunmehr nach außen gestülpte Innenfläche der Handschuhe 16. Man kann, um ein direktes Anströmen der nach außen umgestülpten Innenflächen der Handschuhe 16 zu erreichen, entweder - abweichend von Figur 9 und 11 - die Öffnungen 50,60,...,24''; usw. entsprechend schräg anbringen. Wenn die Öffnungen entsprechend nahe am Rand der Handschuhe gelegen sind, ergibt sich eine gewisse Neigung der Strömung bereits aufgrund des Coanda-Effektes. Auf jeden Fall wird dabei die nach außen gestülpte Innenfläche der Handschuhe 16 schnell getrocknet. Dies ist sogar dann der Fall, wenn man auf die Einschaltung der Heizung verzichtet, wenngleich dies natürlich den Trockenvorgang beschleunigt.

### Bezugszeichenliste

- 1: Wagen
- 2: Vertikale Streben
- 3: Obere horizontale Platte
- 4: Untere horizontale Platte
- 5,6: Kammern
- 7,8: Sauggebläse
- 9,10: Heizungen
- 11: Schalter
- 12,13: Abluftleitungen von 7,8
- 12',13': Warmluftaustrittsrohre (Enden von 12,13)
- 14: Fußschalter
- 15: Ring
- 16: Handschuh
- 17: Wulst von 16
- 18: Auflagefläche von 16 an 21
- 19,20: Öffnungen in 3
- 21: "weitere" Platte
- 22,23: Öffnungen in 21
- 24: Niederhalteplatte
- 24': Rand von 24
- 24'': Öffnungen in 24
- 25: Leiste
- 30: Deckel
- 31: Hebel
- 32: Rändelschraube
- 33: Hebel
- 34: Rändelschraube
- 35: Hebel
- 36: Platte
- 37: Drehverbindung
- 40: Nocken
- 40': schiefe Flächen
- 41: Stift
- 42: Pfeil
- 50-56: Öffnungen in 21
- 57: Schlitz in 21
- 60-66: Öffnungen in 3
- 71: Schalter
- 72: Strömungsumlenkfläche
- 73: Winkel
- 74: Federn
- 75: Handgriffe
- 78: Anschläge
- 80: Bereich zwischen 30 und 21
- 81,82: Infrarotlichtschranke
- 83: Zeitschaltuhr

### Fortsetzung Bezugszeichenliste

- 100: Wagen
- 102: Seitenplatten
- 103: obere horizontale Platte
- 104: untere horizontale Platte
- 105: Kammer
- 107: umschaltbares Gebläse
- 119,120: Öffnungen in 103
- 124: Niederhalteplatte
- 124': Rand von 124
- 125: Niederhalter
- 126: Flansch
- 127: Gewindestange
- 128: Elektromotor
- 130,130': Luftdüsen
- 131: Kammer
- 132: Öffnungen
- 133: Ventilklappen
- 140: Elektromotor
- 141: Niederhalter
- 142: Elektromotor
- 143: Gewindestange
- 144: Flansch
- 183: Steuerung

## Patentansprüche

1. Vorrichtung zum Anziehen und Ausziehen von Handschuhen (16) aus elastischem Material, die zumindest eine mit Öffnungen (19,20;22,23) versehene Kammer (5,6,105) aufweist, und bei der die Öffnungen zum Einbringen der Handschuhe (16) und zum Anlegen derselben mit einer im Manschettenbereich vorgesehenen Auflagefläche (18) beim Ausziehen dienen, und bei der die Kammern (5,6,105) mit Einrichtungen (7,8) zur Erzeugung eines Über- oder Unterdrucks versehen sind, **gekennzeichnet durch** eine Einrichtung (7,8,12,12',13,13',30; 130,130';55,65,24'',51,61) zur Trocknung der Innenflächen der Handschuhe (16) durch Anströmen der Innenflächen mit einer Strömung, die von den genannten Einrichtungen (7,8) zur Erzeugung eines Über- oder Unterdrucks erzeugt wird.

2. Vorrichtung nach Anspruch 1, dadurch gekennzeichnet, daß die Einrichtung durch eine Luftzuführeinrichtung (9,10,12,12',13,13',30) gebildet wird, die in eine Position schwenkbar ist, in der Luftaustrittsrohre (12',13') in den Innenraum der durch von außen aufliegenden Unterdruck gehaltenen Handschuhe (16,16') hineinragen und die aus den Luftaustrittsrohren austretende Luft die Innenflächen der Handschuhe von innen ausströmt.

3. Vorrichtung nach Anspruch 1, dadurch gekennzeichnet, daß die Einrichtungen zur Erzeugung eines Unterdrucks Sauggebläse (7,8) aufweisen.

4. Vorrichtung nach Anspruch 3, dadurch gekennzeichnet, daß die Abluft der Sauggebläse (7,8) in Heizungen (9,10) aufwärmbar ist und der Luftzufuhreinrichtung als Warmluftversorgung zugeführt wird.

5. Vorrichtung nach Anspruch 4, dadurch gekennzeichnet, daß die Warmluftzuführ-Einrichtung durch einen die Öffnungen (19,20;22,23) überdeckenden Deckel (30) gebildet wird, in dem die Warmluftaustrittsrohre (12',13') angeordnet sind.

6. Vorrichtung nach Anspruch 5, dadurch gekennzeichnet, daß die Warmluftaustrittsrohre (12',13') durch die Enden der Abluftleitungen (12,13) der Sauggebläse (7,8) gebildet werden, die den Deckel (30) durchsetzen.

7. Vorrichtung nach Anspruch 6, dadurch gekennzeichnet, daß der Deckel (30) durch Anschläge (73) in einem gewissen Abstand von den Öffnungen (19,20,22,23) zum Einbringen der Handschuhe (16) gehalten wird.

8. Vorrichtung nach Anspruch 5, dadurch gekennzeichnet, daß die Warmluftzuführ-Einrichtung (30) mit der Schwenkbewegung in die genannte Position, in der die Warmluftaustrittsrohre (12',13') in den Innenraum der Handschuhe (16) hineinragen, weitere Öffnungen (60-66) freigibt, die eine Verbindung des Innenraums der Kammern (5,6) mit dem Bereich (80) zwischen den Kammern (5,6) und dem Deckel (30) herstellen, und daß die von den Warmluftaustrittsrohren (12',13') in die Handschuhe (16) eingeblasene Warmluft aus den Handschuhen (16) in diesen Bereich (80) und von diesem in die Kammern (5,6) strömt.

9. Vorrichtung nach Anspruch 8, dadurch gekennzeichnet, daß die Innenseite des Deckels (30) als konkav gewölbte Strömungsumlenkfläche (72) ausgebildet ist.

10. Vorrichtung nach Anspruch 5, dadurch gekennzeichnet, daß über einer die Oberseite der Kammern (5,6) bildenden Platte (3) eine weitere Platte (21) verschiebbar angeordnet ist, in der ebenfalls Öffnungen (22,23) für die Handschuhe (16) sowie weitere Öffnungen (50-56) vorgesehen sind, und daß die weitere Platte (21) derart verschiebbar ist, daß die in ihr vorgesehenen weiteren Öffnungen (50-56) mit den weiteren Öffnungen (60-66) in der die Oberseite der Kammern (5,6) bildenden Platte (3) fluchten.

11. Vorrichtung nach Anspruch 10, dadurch gekennzeichnet, daß die weiteren Öffnungen (50-56,60-66) in der weiteren Platte (21) um die Öffnungen (19,20,22,23) für die Handschuhe (16) derart angeordnet sind, daß durch sie hindurch die nach oben außen umgestülpten Innenflächen der Handschuhe (16) bei Überdruck in der Kammer (105) angeströmt werden.

12. Vorrichtung nach Anspruch 11, dadurch gekennzeichnet, daß die genannten weiteren Öffnungen (50-56,60-66) zu den Öffnungen (19,20) für die Handschuhe (16) hin schräg geneigt sind.

13. Vorrichtung nach Anspruch 10, dadurch gekennzeichnet, daß am Deckel (30) ein Stift (41) angeordnet ist, der bei der Herabbewegung mit einem Nocken (40) zusammenwirkt, der an der weiteren Platte (21) befestigt ist und diese verschiebt.

14. Vorrichtung nach Anspruch 4, dadurch gekennzeichnet, daß mit der Schwenkbewegung der Warmluftzufuhr-Einrichtung (9,10,12,12',13,13',30) ein Schalter (11) betätigt wird, der die Heizungen (9,10) der Warmluftzufuhr-Einrichtung einschaltet.

15. Vorrichtung nach Anspruch 1, dadurch gekennzeichnet, daß durch einen Ring (15) zur Verstärkung des Handschuhs (16) im Manschettenbereich.

16. Vorrichtung nach Anspruch 10, dadurch gekennzeichnet, daß Anschläge (78) vorgesehen sind, die die Lage der durch einen Ring (15) im Manschettenbereich verstärkten Handschuhe (16) auf dem Randbereich der Öffnungen (19,20) festlegen.

17. Vorrichtung nach Anspruch 4, dadurch gekennzeichnet, daß die Warmluftaustrittsrohre (12',13') elastisch sind.

18. Vorrichtung nach Anspruch 1, dadurch gekennzeichnet, daß oberhalb der zum Einbringen der Handschuhe vorgesehenen Öffnungen (19,20;22,23) eine Niederhalteplatte (24) angeordnet ist, deren Rand (24') beim Ausziehen der Handschuhe (16) einen im Manschettenbereich der Handschuhe (16) eingesetzten starren Ring (15) niederhält.

19. Vorrichtung nach Anspruch 1, dadurch gekennzeichnet, daß oberhalb der zum Einbringen der Handschuhe vorgesehenen Öffnungen (19,20;22,23) verschiebbar Niederhalter (125,141) angeordnet sind, die beim Ausziehen der Handschuhe (16) einen im Manschettenbereich der Handschuhe angeordneten Verstärkungsring (15) niederhalten.

20. Vorrichtung nach Anspruch 3 oder einem der folgenden, gekennzeichnet duch eine Lichtschranke (18,82) zum Einschalten der Sauggebläse (7,8) bei Annäherung der Hände (H) an die Öffnungen (19,20;22,23), die in den Kammern (5,6) vorgesehen sind.

21. Vorrichtung nach Anspruch 2 oder einem der folgenden, gekennzeichnet durch eine automatische Abschaltung der Luftzuführ-Einrichtung nach einer bestimmten Zeit.

22. Vorrichtung nach Anspruch 2, dadurch gekennzeichnet, daß die genannten Einrichtung (107) zur Erzeugung des Über- oder Unterdrucks zunächst zur Unterstützung des Ausziehens der Handschuhe (16) Unterdruck erzeugt, daß sie anschließend Überdruck (107) erzeugt, der ein Umstülpen der Handschuhe (16) mit der Innenfläche nach außen bewirkt, und daR dann ein Anströmen der nach außen umgestülpten Innenfläche der Handschuhe (16) durch die Luftzuführ-Einrichtung (130,130') erfolgt.

23. Vorrichtung nach Anspruch 22, dadurch gekennzeichnet, daß die Einrichtung zur Erzeugung von Über- oder Unterdruck ein umschaltbares Sauggebläse (107) ist.

24. Vorrichtung nach Anspruch 22, dadurch gekennzeichnet, daß eine weitere Kammer (130) vorgesehen ist, die mit der erstgenannten Kammer (105) über Ventilklappen (133) in Verbindung steht, die öffnen, wenn in der erstgenannten Kammer (105) Überdruck herrscht, und schließen, wenn in der erstgenannten Kammer Unterdruck herrscht.

25. Vorrichtung nach Anspruch 22, dadurch gekennzeichnet, daR über einer die Oberseite der Kammer (5) bildenden Platte (3) eine weitere Platte (21) verschiebbar angeordnet ist, in der ebenfalls Öffnungen (22,23) für die Handschuhe (16) sowie weitere Öffnungen (50-56) vorgesehen sind, und daß die weitere Platte (21) derart verschiebbar ist, daß die in ihr vorgesehenen weiteren Öffnungen (50-56) mit den weiteren Öffnungen (60-66) in der die Oberseite der Kammern (5,6) bildenden Platte (3) fluchten, und daß die weiteren Öffnungen (50-56,60-66) um die Öffnungen (19,20,22,23) für die Handschuhe (16) derart angeordnet sind, daß durch sie hindurch die Innenfläche der in Folge Überdrucks in der Kammer nach außen umgestülpten Handschuhe angeströmt werden.

26. Vorrichtung nach Anspruch 2, gekennzeichnet durch eine Steuerung (183), die nacheinander folgende Vorgänge betätigt:
(a) Unterdruck in der Kammer (105);
(b) Betätigung von Niederhaltern (124,141) zur Niederhaltung der Manschettenbereiche der Handschuhe (16);
(c) Überdruck in der Kammer (105) bei gleichzeitiger Betätigung der Luftzuführ-Einrichtungen (130,130';55,65,51,61).

## Claims

1. Device for pulling on and pulling out gloves (16) of elastic material, which is provided with at least one chamber (5, 6, 105) having openings (19, 20; 22, 23), said openings serving to introduce the gloves (16) and to position the gloves with their abutment surfaces when pulling them out, said abutment surfaces being provided in the cuff area (18) of the gloves, the chambers (5, 6, 105) being provided with means (7, 8) to generate positive pressure or negative pressure, **characterized by** further means (7, 8, 12, 12', 13, 13', 30'; 130, 130', 55, 65, 24'', 51, 61) for drying the interior surfaces of the gloves (16) by directing a stream to the interior surfaces, said stream being generated by said means (7, 8) for generating positive or negative pressure.

2. Device in accordance to claim 1, characterized in that said means are provided by air supply means (9, 10, 12, 12', 13, 13', 30), which are pivotable into a position, in which air supply tubes (12', 13') project into the interior space of the gloves (16, 16'), said gloves being held by negative pressure applied from the outside, and in which the air supplied by the air supply tubes streams to the interior surfaces of the gloves from the inside.

3. Device in accordance to claim 1, characterized in that the means for generating negative pressure are provided by a suction blower (7, 8).

4. Device in accordance to claim 3, characterized in that the air expelled by the suction blower (7, 8) can be warmed up in heating means (9, 10) and the air supply device is heatable in heaters and is supplied to the air supply means to supply warm air thereto.

5. Device in accordance to claim 4, characterized in that the warm air supply means are provided by a cover (30) covering said openings (19, 20; 22, 23) and in that the warm air supply tubes (12', 13') are provided in the cover.

6. Device in accordance to claim 5, characterized in that the warm air supply tubes (12', 13') are provided by the ends of tubes (12, 13), through which the air is flowing from the suction blower (7, 8), the tubes extending through the cover (30).

7. Device in accordance to claim 6, characterized in that the cover (30) is held by abutment members (23) at a certain distance from the openings (19, 20, 22, 23) for introducing the gloves (16).

8. Device in accordance to claim 5, characterized in that the warm air supply means (30), when being pivoted into said position, in which the warm air supplied tubes (12', 13') project into the interior space of the gloves (16), gives way to further openings (60 to 66), which establish a connection of the interior of said chambers (5, 6) with the area (80) between the chambers (5, 6) and the cover (30), and that the warm air blown by the warm air supply tubes (12', 13') into the gloves (16) flows out of the gloves (16) into this area (80) and from it into the chambers (5, 6).

9. Device in accordance to claim 8, characterized in that the interior side of the cover (30) is formed as a concavely rounded stream deflecting surface (72).

10. Device in accordance to claim 5, characterized in that a further plate (21) is slidably provided above the plate (3), which forms the upper side of the chambers (5, 6), and that said further plate (21) also is provided with openings (22, 23) for the gloves (16) and with further openings, and that the further plate (21) is slidable in such a manner that the further openings (50 to 60) provided therein are in alignment with the further openings (60 to 66) in the plate (3) forming the upper side of the chambers (5, 6).

11. Device in accordance to claim 10, characterized in that the further openings (50 - 56, 60 - 66) in the further plate (21) are provided in such a manner to surround the openings (19, 20, 22, 23) for the gloves (16) that through said openings the outwardly overturned interior surfaces of the gloves (16) are subject to the stream when positive pressure exists in the chamber (105).

12. Device in accordance to claim 11, characterized in that the said further openings (15 - 56, 60 - 66) are inclined into the direction of the openings (19, 20) for the gloves (16).

13. Device in accordance to claim 10, characterized in that a pin (41) is provided at the cover (30), said pin when being moved downwardly cooperating with a cam (40) connected to the further plate (21) displacing the same.

14. Device in accordance to claim 4, charcterized in that with the pivot movement of the warm air supply means (9, 10, 12, 12', 13, 13', 30) a switch (11) is operated, which switches on the heating means (9, 10) of the warm air supply means.

15. Device in accordance to claim 1, characterized by a ring (15) for strenghtening the glove (16) in the cuff area.

16. Device in accordance to claim 10, characterized in that abutment members (28, 70) are provided, which determine the position of the gloves (16), the gloves being strengthened in their cuff area by a ring (15), the position being at the edge area of the openings (19, 20).

17. Device in accordance to claim 4, characterized in that the warm air supply tubes (12', 13') are elastic.

18. Device in accordance to claim 1, characterized in that above the openings (19, 20; 22, 23) provided for introducing the gloves, a holding down plate (24) is provided, the edge (24) of which upon gloves (16) being pulled out holds a rigid ring (15) inserted in the cuff area of the gloves (16).

19. Device in accordance to claim 1, characterized in that above of the openings (19, 20; 22, 23) provided for introducing the gloves, slidable holding down means (125, 141) are provided, which, upon pulling out the gloves (16), hold down an strenghtening ring (15) provided in the cuff area of the gloves.

20. Device in accordance to claim 3 or one of the following, characterized in that a light barrier (18, 22) is provided for switching on the suction blower (7, 8) upon the hands (H) coming closer to the openings (19, 20; 22, 23) in the chambers (5, 6).

21. Device in accordance to claim 2 or one of the following, characterized by automatic switching off of the air supply means after a certain time.

22. Device in accordance to claim 2, characterized in that the said means (107) for generating the positive or negative pressure at first provides negative pressure for supporting the pulling out of the gloves (16), that it subsequently produces positive pressure (107), which effects a overturning of the gloves (16) with their interior surface to the outside, and that thereafter a flow is provided onto the outwardly overturned interior surface of the gloves (16) by the air supply means (130, 130').

23. Device in accordance to claim 22, characterized in that the device for generating positive or negative pressure is a reversable suction blower (107).

24. Device in accordance to claim 22, characterized in that a further chamber (130) is provided, connected to the first mentioned chamber (105) by means of valve flaps (133), which open, when there is positive pressure in the first mentioned chamber (105), and which close, when there is negative pressure in the first mentioned chamber.

25. Device in accordance to claim 22, characterized in that above the plate (3) forms the upper side of the chamber (5) a further plate (21) is slidably provided, in which also openings (22, 23) for the gloves and further openings (50 - 56) are provided and that the further plate (21) is slidable in such a manner that the further openings (50 - 56) provided therein are in alignment with the further openings (60 - 66) in the plate (3) forming the upper side of the chambers (5, 6), and that the further openings (50 - 56, 60 - 66) are provided to surround the openings (19, 20, 22, 23) for the gloves (16) in such a manner, that through the same flow is directed to the interior surface of the gloves overturned outwardly as a consequence of the positive pressure in the chamber.

26. Device in accordance to claim 2, characterized by a control means (183) effecting in sequence the following steps:
(a) negative pressure in the chamber (105);
(b) operation in the down holding means (124, 141) for holding down the cuff areas of the gloves (16);
(c) positive pressure in the chamber (105) and simultaneous operation of the air supply means (130, 130'; 55, 65, 51, 61).

## Revendications

1. Dispositif servant à mettre ou à retirer des gants (16) en matière élastique, qui comprend au moins une chambre (5, 6, 105), munie d'ouvertures (19, 20 ; 22, 23), et dans laquelle les ouvertures servent à introduire les gants (16), afin de les mettre, et comprennent une surface d'appui (18) prévue dans la zone du poignet pour retirer lesdits gants, et dans lequel les chambres (5, 6, 105) sont munies de systèmes (7, 8) destinés à produire une surpression et une dépression, caractérisé en ce qu'un système (7, 8, 12, 12', 13, 13', 30 ; 130, 130' ; 55, 65, 24'', 51, 61) destiné au séchage des surfaces intérieures des gants (16) par le soufflage d'un courant sur les surfaces intérieures, lequel est produit par les systèmes cités (7, 8), destinés à produire une surpression et une dépression.

2. Dispositif selon la revendication 1, caractérisé en ce que le système est constitué par un système d'admission d'air (9, 10, 12, 12', 13, 13', 30), qui peut être pivoté dans une position dans laquelle les tubes d'évacuation d'air (12', 13') pénètrent dans l'espace intérieur des gants (16, 16'), maintenu par la dépression appliquée de l'extérieur, et en ce que l'air évacué par les tubes d'évacuation d'air provoque un soufflage de l'intérieur vers l'extérieur des surfaces intérieures des gants.

3. Dispositif selon la revendication 1, caractérisé en ce que les systèmes destinés à produire une dépression sont munis d'un ventilateur aspirant (7, 8).

4. Dispositif selon la revendication 3, caractérisé en ce que l'air évacué par le ventilateur aspirant (7, 8) peut être réchauffé dans des appareils de chauffage (9, 10) et amené sous forme d'air chaud dans le système d'admission d'air.

5. Dispositif selon la revendication 4, caractérisé en ce que le système d'admission d'air chaud est formé par un couvercle (30), qui recouvre les ouvertures (19, 20 ; 22, 23), dans lequel sont montés les tubes d'évacuation d'air chaud (12', 13').

6. Dispositif selon la revendication 5, caractérisé en ce que les tubes d'évacuation d'air chaud (12', 13') sont formés par les extrémités des conduites d'évacuation d'air (12, 13) du ventilateur aspirant (7, 8), qui traversent le couvercle (30).

7. Dispositif selon la revendication 6, caractérisé en ce que le couvercle (30) est maintenu, par l'intermédiaire de butées (73), à une distance déterminée des ouvertures (19, 20, 22, 23), dans lesquelles on introduit les gants (16).

8. Dispositif selon la revendication 5, caractérisé en ce que le système d'admission d'air chaud (30) est amené par un mouvement de pivotement dans la position mentionnée, dans laquelle les tubes d'évacuation d'air chaud (12', 13') pénètrent dans l'espace intérieur des gants (16), débloquent des ouvertures supplémentaires (60-66), par lesquelles l'espace intérieur des chambres (5, 6) communique avec la zone (80) située entre les chambres (5, 6) et le couvercle (30), et en ce que l'air chaud insufflé dans les gants (16) par les tubes d'évacuation d'air chaud (12', 13') s'échappe des gants (16) pour circuler dans cette zone (80) et à partir de celle-ci dans les chambres (5, 6).

9. Dispositif selon la revendication 8, caractérisé en ce que la face intérieure du couvercle (30) forme une surface de guidage du courant (72) à courbure concave.

10. Dispositif selon la revendication 5, caractérisé en ce que sur une plaque (3), formant la paroi supérieure des chambres (5, 6), est montée une plaque supplémentaire (21), susceptible d'être déplacée, qui est également munie d'ouvertures (22, 23) pour les gants (16), ainsi que d'ouvertures supplémentaires (50-56), et en ce que la plaque supplémentaire (21) peut être déplacée, de telle sorte que les ouvertures supplémentaires (50-56) réalisées dans cette dernière s'alignent avec les ouvertures supplémentaires (60-66) réalisées dans la plaque (3) formant la paroi supérieure des chambres (5, 6).

11. Dispositif selon la revendication 10, caractérisé en ce que les ouvertures supplémentaires (50-56, 60-66) dans la plaque supplémentaire (21) sont disposées autour des ouvertures (19, 20, 22, 23) pour les gants (16), de telle sorte que les surfaces intérieures des gants (16), retournées vers l'extérieur vers le haut, sont soufflées à travers lesdites ouvertures pour gants en cas de surpression dans la chambre (105).

12. Dispositif selon la revendication 11, caractérisé en ce que les ouvertures supplémentaires (50-56, 60-66) mentionnées sont inclinées en biais par rapport aux ouvertures (19, 20) pour les gants (16).

13. Dispositif selon la revendication 10, caractérisé en ce que le couvercle (30) est muni d'une pointe (41), qui, lors d'un pivotement vers le bas, entre en action avec une pièce saillante (40), fixée sur la plaque supplémentaire (21), et déplace cette dernière.

14. Dispositif selon la revendication 4, caractérisé en ce que le mouvement de pivotement du système d'admission d'air chaud (9, 10, 12, 12', 13, 13', 30) actionne un interrupteur (11) qui branche les appareils de chauffage (9, 10) du système d'admission d'air chaud.

15. Dispositif selon la revendication 1, caractérisé en ce qu'un anneau (15) est utilisé pour renforcer le gant (16) dans la zone du poignet.

16. Dispositif selon la revendication 10, caractérisé en ce qu'il est prévu de monter des butées (78), qui définissent la position des gants (16), renforcés par un anneau (15) dans la zone des poignets, sur le bord des ouvertures (19, 20).

17. Dispositif selon la revendication 4, caractérisé en ce que les conduites d'évacuation d'air chaud (12', 13') sont des tuyaux flexibles.

18. Dispositif selon la revendication 1, caractérisé en ce qu'au-dessus des ouvertures (19, 20 ; 22, 23), dans lesquelles on introduit les gants, est montée une plaque de blocage (24), dont le bord (24') bloque un anneau rigide (15), monté dans la zone des poignets des gants (16), au moment où l'on retire les gants (16).

19. Dispositif selon la revendication 1, caractérisé en ce qu'au-dessus des ouvertures (19, 20 ; 22, 23), dans lesquelles on introduit les gants, sont montés des systèmes de blocage (125, 141), susceptibles d'être déplacés, qui bloquent un anneau de renforcement (15), monté dans la zone des poignets des gants (16), au moment où l'on retire les gants (16).

20. Dispositif selon la revendication 3, caractérisé par un barrage photoélectrique (18, 82) destiné à brancher le ventilateur aspirant (7, 8) dès que les mains (H) arrivent à proximité des ouvertures (19, 20 ; 22, 23), qui sont prévues dans les chambres (5, 6).

21. Dispositif selon la revendication 2 ou l'une des revendications suivantes, caractérisé par un débranchement automatique du système d'admission d'air après un temps détermine.

22. Dispositif selon la revendication 2, caractérisé en ce que le système (107) mentionné, destiné à produire la surpression ou la dépression, produit d'abord une dépression pour aider à retirer les gants (16), en ce qu'il produit ensuite une surpression (107) qui provoque un relèvement des gants (16) de manière à exposer leur surface intérieure à l'extérieur, et en ce que le système d'admission d'air (130, 130') souffle alors sur la surface intérieure des gants (16), retournée vers l'extérieur.

23. Dispositif selon la revendication 22, caractérisé en ce que le système destiné à produire une surpression et une dépression est un ventilateur aspirant (107) réversible.

24. Dispositif selon la revendication 22, caractérisé en ce qu'il est prévu de monter une chambre supplémentaire (130), qui communique avec la chambre (105), citée en premier lieu, par des clapets de soupape (133), qui ouvrent lorsqu'il règne une surpression dans la chambre (105), citée en premier lieu, et ferment lorsqu'il règne une dépression dans la chambre citée en premier lieu.

25. Dispositif selon la revendication 22, caractérisé en ce que sur une plaque (3), formant la paroi supérieure de la chambre (5), est montée une plaque supplémentaire (21), susceptible d'être déplacée, qui est également munie d'ouvertures (22, 23) pour les gants (16), ainsi que d'ouvertures supplémentaires (50-56), et en ce que la plaque supplémentaire (21) peut être déplacée, de telle sorte que les ouvertures supplémentaires (50-56) réalisées dans cette dernière s'alignent avec les ouvertures supplémentaires (60-66) réalisées dans la plaque (3), formant la paroi supérieure des chambres (5, 6), et en ce que les ouvertures supplémentaires (50-56, 60-66) sont disposées autour des ouvertures (19, 20, 22, 23) pour les gants (16), de telle sorte que les surfaces intérieures des gants, retournées à l'extérieur vers le haut, sont soufflées à travers lesdites ouvertures en cas de surpression.

26. Dispositif selon la revendication 2, caractérisé par un système de commande (183), qui actionne successivement les processus suivants :
(a) dépression dans la chambre (105) ;
(b) actionnement des systèmes de blocage (124, 141) destinés à bloquer les zones des poignets des gants (16) ;
(c) surpression dans la chambre (105) et, simultanément, actionnement des systèmes d'admission d'air (130, 130' ; 55, 65, 51, 61).
